# EUROPEAN PATENT APPLICATION

(11) **EP 4 721 726 A1**
(43) Date of publication of application: **08.04.2026**
(21) Application number: 24815904.8
(22) Date of filing: 31.05.2024
(51) Int. Cl.: A61K 9/10, A61K 47/44, A61K 47/10, A61K 9/16, A61K 9/00, A61K 38/26, A61P 3/10, A61K 47/14

(54) **PHARMACEUTICAL COMPOSITION COMPRISING GLP-1 RECEPTOR AGONIST, GIP/GLP-1 RECEPTOR AGONIST AND/OR GLP-1/GIP/GCG RECEPTOR TRIPLE AGONIST**

(30) Priority: 02.06.2023 KR 20230071862; 16.02.2024 KR 20240022879
(71) Applicant: Aulbio Co., Ltd., Seongnam-si, Gyeonggi-do 13105 (KR)
(72) Inventor: KIM, Cherng Ju, Beaumont, California 92223 (US); AN, Tae Kun, Yongin-si, Gyeonggi-do 16990 (KR); KIM, A Ram, Seoul 05667 (KR); KIM, Seo A, Suwon-si, Gyeonggi-do 16509 (KR); PARK, Ji Won, Hwaseong-si, Gyeonggi-do 18266 (KR)
(74) Representative: dompatent
(86) International application number: PCT/KR2024/007467
(87) International publication number: WO 2024/248530

(57) **Abstract**

The present invention provides a pharmaceutical composition, comprising: microspheres containing one or more selected from a GLP-1 receptor agonist, a GIP/GLP-1 receptor agonist and a GLP-1/GIP/GCG receptor triple agonist; and one or more pharmaceutically acceptable first carriers selected from the group consisting of a fluid oil containing a fatty acid ester, a surfactant and a polyhydric alcohol. The microspheres of the pharmaceutical composition remain stable for a long period of time in a suspension formulation, and the microspheres stored for a long period of time exhibit excellent dissolution characteristics. In addition, the pharmaceutical composition exhibits the effects of increasing bioavailability and reducing the dosage to be administered.

## Description

### [Technical Field]

The present invention relates to a pharmaceutical composition comprising a GLP-1 receptor agonist, a GIP/GLP-1 receptor agonist and/or a GLP-1/GIP/GCG receptor triple agonist.

### [Background Art]

Glucagon-like peptide-1 (GLP-1) is derived from preproglucagon, which is a 158-amino acid precursor polypeptide that is processed in different tissues to form a number of different proglucagon-derived peptides, including glucagon, glucagon-like peptide-1 (GLP-1), glucagon-like peptide-2 (GLP-2) and oxyntomodulin (OXM), that are involved in a wide variety of physiological functions, including glucose homeostasis, insulin secretion, gastric emptying and intestinal growth, as well as the regulation of food intake.

GLP-1 is produced as a 37-amino acid peptide that corresponds to amino acids 72-108 of proglucagon (92-128 of preproglucagon). GLP-1(7-36) amide or GLP-1(7-37) acid are biologically active forms of GLP-1 that demonstrates essentially equivalent activity at the GLP-1 receptor. GLP-1 and GLP-1 analogues, which act as agonists at the GLP-1 receptor, have been found to provide, for example, effective glycemic control for the treatment of patients with type 2 diabetes, and additionally provide body weight loss effects, preserved beta-cell function, and ameliorated hypertension, hypoglycemia and/or hyperlipidemia.

Currently, certain GLP-1 analogues, including Byetta^{®} and Bydureon BCise^{®} (exenatide), Ozempic^{®} (semaglutide), Victoza^{®} (liraglutide), Adlyxin^{®} (lixisenatide), Tanzeum^{®} (albiglutide) and Trulicity^{®} (dulaglutide), are commercially available or under development.

Like GLP-1, gastric inhibitory polypeptide (GIP) not only stimulates insulin secretion in beta cells in a glucose-dependent manner, but also promotes insulin synthesis, induces beta cell proliferation, and inhibits apoptosis [Trumper et al., Mol Endocrinol 15:1559-1570, 2001].

Currently, a GLP-1/GIP dual agonist is also being developed, and for example, Mounjaro^{®} (tirzepatide) is commercially available.

GCG receptors regulate the action of glucagon in the liver. Glucagon plays a role in increasing blood glucose levels by promoting gluconeogenesis in the liver, and GCG receptors help maintain blood glucose levels at a constant level by inhibiting the action of glucagon. Currently, a GLP-1/GIP/GCG receptor triple agonist is being developed.

The GLP-1 receptor agonist, GLP-1/GIP receptor agonist and/or GLP-1/GIP/GCG receptor triple agonist are primarily administered as injections due to several barriers, such as enzymatic degradation in the gastrointestinal tract and intestinal mucosa, insufficient absorption from the intestinal mucosa, and first-pass metabolism in the liver.

An injectable sustained-release formulation of the GLP-1 receptor agonist, GLP-1/GIP receptor agonist and/or GLP-1/GIP/GCG receptor triple agonist may provide a therapeutic amount of the active ingredient over a long period of time in a single injection, and therefore, it is not necessary to inject daily. Commonly used injectable sustained-release formulations are prepared, for example, to comprise microspheres and aqueous carriers.

However, the microspheres do not have long-term stability in the aqueous carriers, and therefore, there is a disadvantage in that the microspheres and the aqueous carriers must be packaged and stored separately. In addition, patients experience significant inconvenience because they must perform several steps to combine the microspheres and the aqueous carriers before injection administration.

### [Prior Art Document]

### [Patent Document]

(Patent Document 1) Korean Laid-open Patent Publication No. 10-2020-0044016

### [Disclosure]

### [Technical Problem]

The present invention has been devised to address the aforementioned problems of the prior art, and
it is an object to provide a pharmaceutical composition, wherein microspheres containing a GLP-1 receptor agonist, a GIP/GLP-1 receptor agonist and/or a GLP-1/GIP/GCG receptor triple agonist may maintain long-term stability in a suspension.

In addition, it is another object to provide the pharmaceutical composition, wherein the microspheres stored for a long period of time in a suspension may exhibit excellent dissolution characteristics.

In addition, it is another object to provide the pharmaceutical composition comprising a surfactant or a polyhydric alcohol as carriers for the microspheres to enhance bioavailability.

### [Technical Solution]

In order to achieve the aforementioned objects,
the present invention provides a pharmaceutical composition comprising: microspheres containing one or more selected from a GLP-1 receptor agonist, a GIP/GLP-1 receptor agonist and a GLP-1/GIP/GCG receptor triple agonist; and
one or more pharmaceutically acceptable first carriers selected from the group consisting of a fluid oil containing a fatty acid ester, a surfactant and a polyhydric alcohol.

In addition, the present invention provides the pharmaceutical composition, wherein the fluid oil is one or more selected from coconut oil, palm oil, palm kernel oil, sesame oil, soybean oil, almond oil, rapeseed oil, corn oil, sunflower oil, peanut oil, olive oil, castor oil, safflower oil, cottonseed oil and ethyl oleate.

In addition, the present invention provides the pharmaceutical composition, wherein the surfactant is one or more selected from poloxamer 188, poloxamer 407, polysorbate 20, polysorbate 60, polysorbate 80, polyoxyethylene oleyl ether, polyoxyethylene cetyl ether, polyoxyethylene stearyl ether and polyoxyethylene lauryl ether.

In addition, the present invention provides the pharmaceutical composition, wherein the polyhydric alcohol is one or more selected from ethylene glycol, propylene glycol, polyethylene glycol, glycerol, erythritol, threitol, xylitol, ribitol, mannitol, sorbitol and maltitol.

In addition, the present invention provides the pharmaceutical composition, wherein the polyhydric alcohol is ethylene glycol, propylene glycol, polyethylene glycol or glycerol.

In addition, the present invention provides the pharmaceutical composition, which comprises one or more selected from the GLP-1 receptor agonist, GIP/GLP-1 receptor agonist and GLP-1/GIP/GCG receptor triple agonist contained in the microspheres in a concentration ranging from 0.01 mg to 2,000 mg based on 1 mL of the first carriers.

In addition, the present invention provides the pharmaceutical composition, wherein the GLP-1 receptor agonist, GIP/GLP-1 receptor agonist and/or GLP-1/GIP/GCG receptor triple agonist are one or more selected from exenatide, liraglutide, lixisenatide, albiglutide, dulaglutide, semaglutide, tirzepatide, cotadutide, taspoglutide, retatrutide and pharmaceutically acceptable salts thereof.

In addition, the present invention provides the pharmaceutical composition, wherein one or more selected from the GLP-1 receptor agonist, GIP/GLP-1 receptor agonist and GLP-1/GIP/GCG receptor triple agonist are contained in an amount of 3 to 50% by weight based on the total weight of the microspheres.

In addition, the present invention provides the pharmaceutical composition, wherein the microspheres further contain a biocompatible polymer.

In addition, the present invention provides the pharmaceutical composition, wherein the biocompatible polymer is one or more selected from polylactic acid, polylactide, polylactic-co-glycolic acid, polylactide-co-glycolide (PLGA), polyphosphazine, polyiminocarbonate, polyphosphoester, polyanhydride, polyorthoester, copolymer of lactic acid and caprolactone, polycaprolactone, polyhydroxyvalerate, polyhydroxybutyrate, polyamino acid and copolymer of lactic acid and amino acid.

In addition, the present invention provides the pharmaceutical composition, wherein the biocompatible polymer is contained in an amount of 50 to 97% by weight based on the total weight of the microparticles.

In addition, the present invention provides the pharmaceutical composition, which further comprises one or more additives selected from a buffer, an isotonic agent, a preservative and a pH adjusting agent.

In addition, the present invention provides the pharmaceutical composition, wherein the microspheres contain one or more selected from the GLP-1 receptor agonist, GIP/GLP-1 receptor agonist and GLP-1/GIP/GCG receptor triple agonist and the biocompatible polymer at a weight ratio of 1:1-50.

In addition, the present invention provides the pharmaceutical composition, wherein the microspheres remain stable for 6 months or more.

In addition, the present invention provides the pharmaceutical composition, which is an injection.

### [Advantageous Effects]

The pharmaceutical composition of the present invention provides the effect that microspheres containing a GLP-1 receptor agonist, a GIP/GLP-1 receptor agonist and/or a GLP-1/GIP/GCG receptor triple agonist maintain long-term stability in a suspension.

In addition, it provides the effect that the microspheres stored for a long period of time in a suspension exhibit excellent dissolution characteristics.

In addition, it provides the effect that the surfactant or polyhydric alcohol according to the present invention may be used as carriers for the microspheres to increase bioavailability and reduce the dosage administered.

### [Description of Drawings]

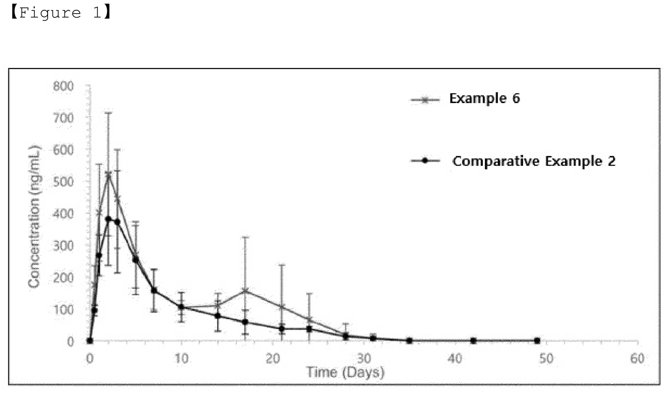

FIG. 1 is a graph showing the *in vivo* pharmacokinetics of semaglutide prepared in Example 6 and Comparative Example 2.

### [Best Mode]

Hereinafter, the present invention will be described in more detail.

Unless defined otherwise, all technical terms used in the present invention have the same meaning as commonly understood by those skilled in the relevant field of the present invention. In addition, those similar or equivalent to the preferred methods or samples described in the present invention are also included in the scope of the present invention. The contents of all publications cited as references in this specification are incorporated herein by reference in their entirety.

The present invention relates to a pharmaceutical composition comprising:
microspheres containing one or more selected from a GLP-1 receptor agonist, a GIP/GLP-1 receptor agonist and a GLP-1/GIP/GCG receptor triple agonist; and
one or more pharmaceutically acceptable first carriers selected from the group consisting of a fluid oil containing a fatty acid ester, a surfactant and a polyhydric alcohol.

A constant challenge for researchers in this field is to improve the ease of administration of a GLP-1 receptor agonist, a GIP/GLP-1 receptor agonist and/or a GLP-1/GIP/GCG receptor triple agonist. To address this challenge, sustained-release microspheres containing these drugs have been developed. The sustained-release microspheres have significantly improved the convenience of administration for patients by drastically reducing the number of injections.

The sustained-release microspheres are suspended in aqueous carriers and injected into the human body in a suspension state. However, there was a problem in that the stability of the sustained-release microspheres in aqueous carriers was low, making it difficult to prepare and distribute them in a suspension formulation. Therefore, the microspheres and the aqueous carriers are currently distributed in a separate state, and medical staff or patients mix them to prepare a suspension formulation before use for injection. Preparing the suspension formulation is not only cumbersome, but also causes problems in that the concentration of the drug cannot be precisely adjusted during the preparation process of the suspension formulation when the patient directly injects it.

The pharmaceutical composition of the present invention has the characteristics that the microspheres containing the GLP-1 receptor agonist, GIP/GLP-1 receptor agonist and/or GLP-1/GIP/GCG receptor triple agonist maintain long-term stability in a suspension, and the microspheres stored for a long period of time exhibit excellent dissolution characteristics.

In addition, the pharmaceutical composition of the present invention has the characteristics that it comprises one or more selected from a fluid oil containing a fatty acid ester, a surfactant and a polyhydric alcohol as first carriers, thereby improving the stability of the microspheres, increasing bioavailability, and reducing the dosage administered.

In one embodiment of the present invention, the fluid oil carrier is a pharmaceutically acceptable carrier and may be non-aqueous or lipophilic. The fluid oil carrier may be substantially inert, so that it does not interact with the microspheres, and non-toxic, so that it does not have a negative effect on the patient. In addition, it may not dissolve the biocompatible polymer forming the microspheres. In addition, it may not dissolve the drug contained within the microspheres.

In addition, microspheres using a fluid oil as carriers may increase bioavailability compared to microspheres not using the fluid oil as carriers.

The "non-aqueous" does not exclude the inclusion of trace amounts of residual water that do not negatively affect the solubility of the microspheres. The oil carrier may not dissolve the microspheres to an extent that would negatively affects the stability of the microspheres or cause a loss of initial release control of the microspheres. In addition, the oil carrier may not swell the microspheres to an extent that would negatively affect the stability of the microspheres.

The fluid oil refers to a substance that is viscous liquid at ambient temperature or slightly higher temperature, and is hydrophobic (immiscible with water) and lipophilic (literally miscible with other oils). As the fluid oil carrier, one or more selected from the group consisting of, for example, coconut oil, palm oil, palm kernel oil, sesame oil, soybean oil, almond oil, rapeseed oil, corn oil, sunflower oil, peanut oil, olive oil, castor oil, safflower oil, cottonseed oil and ethyl oleate may be used.

The fluid oil may be medium-chain triglycerides (MCT) oil. The MCT oil refers to an oil produced by isolating and refining only the medium-chain fatty acids contained in fats and oils.

In one embodiment of the present invention, microspheres using a surfactant or a polyhydric alcohol as carriers may increase bioavailability by 10% or more compared to microspheres not using the surfactant or the polyhydric alcohol as carriers.

In one embodiment of the present invention, the surfactant may be one or more selected from poloxamer 188, poloxamer 407, polysorbate 20, polysorbate 60, polysorbate 80, polyoxyethylene oleyl ether, polyoxyethylene cetyl ether, polyoxyethylene stearyl ether, and polyoxyethylene lauryl ether. More specifically, the surfactant may be poloxamer 188, poloxamer 407, polyoxyethylene oleyl ether, polyoxyethylene cetyl ether, polyoxyethylene stearyl ether or polyoxyethylene lauryl ether, but is not limited to these examples.

In one embodiment of the present invention, the polyhydric alcohol may be one or more selected from ethylene glycol, propylene glycol, polyethylene glycol, glycerol, erythritol, threitol, xylitol, ribitol, mannitol, sorbitol and maltitol. More specifically, the polyhydric alcohol may be ethylene glycol, propylene glycol, polyethylene glycol or glycerol, but is not limited to these examples.

In one embodiment of the present invention, the pharmaceutical composition may comprise one or more selected from the GLP-1 receptor agonist, GIP/GLP-1 receptor agonist and GLP-1/GIP/GCG receptor triple agonist contained in the microspheres in a concentration ranging from 0.01 mg to 2,000 mg based on 1 mL of the first carriers. In this case, the first carriers suspend one or more selected from the GLP-1 receptor agonist, GIP/GLP-1 receptor agonist and GLP-1/GIP/GCG receptor triple agonist included in the microspheres, and the pharmaceutical composition may comprise one or more selected from the GLP-1 receptor agonist, GIP/GLP-1 receptor agonist and GLP-1/GIP/GCG receptor triple agonist suspended in an amount of 0.01 mg to 2,000 mg based on 1 mL of the first carrier.

More specifically, the concentration of the pharmaceutical composition may be 0.01 mg or more, 0.05 mg or more, 0.1 mg or more, 0.15 mg or more, 0.2 mg or more, 0.25 mg or more, 0.3 mg or more, 0.35 mg or more, 0.4 mg or more, 0.45 mg or more, 0.5 mg or more, 0.55 mg or more, 0.6 mg or more, 0.65 mg or more, 0.7 mg or more, 0.75 mg or more, 0.8 mg or more, 0.85 mg or more, 0.9 mg or more, 0.95 mg or more or 1.0 mg or more, or may be 2,000 mg or less, 1,995 mg or less, 1,990 mg or less, 1,985 mg or less, 1,980 mg or less, 1,975 mg or less, 1,970 mg or less, 1,965 mg or less, 1,960 mg or less, 1,955 mg or less, 1,950 mg or less, 1,945 mg or less, 1,940 mg or less, 1,935 mg or less, 1,930 mg or less, 1,925 mg or less, 1,920 mg or less, 1,915 mg or less, 1,910 mg or less, 1,905 mg or less or 1,900 mg or less, of one or more selected from the GLP-1 receptor agonist, GIP/GLP-1 receptor agonist and/or GLP-1/GIP/GCG receptor triple agonist contained in the microspheres, based on 1 mL of the first carriers, but is not limited to these ranges.

The pharmaceutical composition of the present invention may consist of microspheres and carriers excluding the microspheres, and the carriers may contain the first carriers in a lower limit of 20% by weight or more, 30% by weight or more, 40% by weight or more, 50% by weight or more, 60% by weight or more, 70% by weight or more, 80% by weight or more, 90% by weight or more or 100% by weight, based on 100% by weight of the carriers. In addition, the first carriers may be contained in an upper limit of 90% by weight or less, 80% by weight or less, 70% by weight or less, 60% by weight or less, 50% by weight or less, 40% by weight or less or 30% by weight or less. In addition, the first carriers may be contained in a range formed by a combination of the lower limit and the upper limit.

In the "pharmaceutical composition consisting of microspheres and carriers excluding the microspheres," the carriers refer to carriers in a broad sense that includes all components used to facilitate administration of the microspheres. For example, additives and the like are also included in the carrier in the broad sense. The carriers may consist of, for example, the first carriers and additives.

In one embodiment of the present invention, one or more selected from the GLP-1 receptor agonist, GIP/GLP-1 receptor agonist and GLP-1/GIP/GCG receptor triple agonist may be selected from the group consisting of one or more selected from exenatide, liraglutide, lixisenatide, albiglutide, dulaglutide, semaglutide, tirzepatide, cotadutide, taspoglutide, retatrutide and pharmaceutically acceptable salts thereof. More specifically, one or more selected from the GLP-1 receptor agonist, GIP/GLP-1 receptor agonist and GLP-1/GIP/GCG receptor triple agonist may be selected from the group consisting of semaglutide, tirzepatide, retatrutide and pharmaceutically acceptable salts thereof.

As the pharmaceutically acceptable salts, salts commonly used in the art may be used without limitation. The term "pharmaceutically acceptable salts" in the present invention refers to any and all organic or inorganic addition salts of the compounds, in which the side effects caused by such salts do not reduce the beneficial efficacies of the active ingredients at a concentration having a relatively non-toxic and harmless effective action on the patient. Specific examples include, but are not limited to, acetate, benzoate, hydroxynaphthoate, napadicylate and pamoate of each of the drugs, and any of various forms of salt known in the art may be included.

One or more selected from the GLP-1 receptor agonist, GIP/GLP-1 receptor agonist and GLP-1/GIP/GCG receptor triple agonist may be prepared in various forms, and for example, may be amorphous or crystalline.

The microspheres according to one embodiment of the present invention mean microspheres in which the GLP-1 receptor agonist, GIP/GLP-1 receptor agonist or GLP-1/GIP/GCG receptor triple agonist is encapsulated in the microsppheres prepared using a biocompatible polymer, and in the present specification, these are simply referred to as microspheres containing a GLP-1 receptor agonist, GIP/GLP-1 receptor agonist or GLP-1/GIP/GCG receptor triple agonist, a GLP-1 receptor agonist, GIP/GLP-1 receptor agonist or GLP-1/GIP/GCG receptor triple agonist microspheres, or microspheres.

In one embodiment of the present invention, the biocompatible polymer means a polymer that has been guaranteed to be safe *in vivo* by not causing high cytotoxicity and inflammatory response when administered into a living body, and is also referred to simply as a polymer in this specification.

In one embodiment of the present invention, the microspheres may further contain a biocompatible polymer. As the biocompatible polymer, any biocompatible polymer known in the art may be used without limitation, and specifically, one or more selected from, for example, polylactic acid, polylactide, polylactic-co-glycolic acid, polylactide-co-glycolide (PLGA), polyphosphazine, polyiminocarbonate, polyphosphoester, polyanhydride, polyorthoester, copolymer of lactic acid and caprolactone, polycaprolactone, polyhydroxyvalerate, polyhydroxybutyrate, polyamino acid, and copolymer of lactic acid and amino acid may be used.

In one embodiment of the present invention, the biocompatible polymer may have an intrinsic viscosity of 0.15 dL/g to 1.7 dL/g, 0.15 dL/g to 1.3 dL/g, or 0.15 dL/g to 1.2 dL/g.

The intrinsic viscosity refers to a value measured at a concentration of 0.05 to 3% (w/v) in chloroform at 25°C using an Ubbelohde viscometer. If the intrinsic viscosity is less than 0.15 dL/g, the molecular weight of the polymer is insufficient, making it difficult to exhibit a sustained-release effect of the drug, and if the intrinsic viscosity exceeds 1.7 dL/g, the effect of excessively delaying the release of the drug may occur. In addition, when preparing microspheres using a polymer with high intrinsic viscosity, there is a problem in that an excessive amount of preparation solvent must be used due to the high viscosity of the polymer, and it is difficult to prepare reproducible microspheres.

Examples of commercially available polymers having said properties include Evonik company's Resomer series of RG502H, RG503H, RG504H, RG502, RG503, RG504, RG653H, RG752H, RG752S, RG755S, RG750S, RG757S, RG858S, R202H, R203H, R205H, R202S, R203S, R205S, R206S and R207S, Corbion company's PDL 02A, PDL 02, PDL 04, PDL 05, PDLG 7502A, PDLG 7502, PDLG 7507, PDLG 5002A, PDLG 5002, PDLG 5004A, PDLG 5004, etc.

In one embodiment of the present invention, the microspheres may further contain one or more release-controlling agents selected from butyric acid, valeric acid, caproic acid, enanthic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, arachidic acid, isocrotonic acid, oleic acid, elaidic acid, sorbic acid, linoleic acid, arachidonic acid, hydroxynaphthoic acid, napadisylic acid and pamoic acid.

In one embodiment of the present invention, one or more selected from the GLP-1 receptor agonist, GIP/GLP-1 receptor agonist and GLP-1/GIP/GCG receptor triple agonist may be contained in an amount of 3 to 50% by weight based on the total weight of the microspheres. More specifically, one or more selected from the GLP-1 receptor agonist, GIP/GLP-1 receptor agonist and GLP-1/GIP/GCG receptor triple agonist may be contained in an amount of 3.0% by weight or more, 3.5% by weight or more, 4.0% by weight or more, 4.5% by weight or more, 5.0% by weight or more, 5.5% by weight or more, 6.0% by weight or more, 6.5% by weight or more, 7.0% by weight or more, 7.5% by weight or more, 8.0% by weight or more, 8.5% by weight or more, 9.0% by weight or more, 9.5% by weight or more or 10.0% by weight or more, or may be 50% by weight or less, 49.5% by weight or less, 49.0% by weight or less, 48.5% by weight or less, 48.0% by weight or less, 47.5% by weight or less, 47.0% by weight or less, 46.5% by weight or less, 46.0% by weight or less, 45.5% by weight or less, 45.0% by weight or less, 44.5% by weight or less, 44.0% by weight or less, 43.5% by weight or less, 43.0% by weight or less, 42.5% by weight or less, 42.0% by weight or less, 41.5% by weight or less, 41.0% by weight or less, 40.5% by weight or less or 40.0% by weight or less, based on the total weight of the microspheres.

One or more selected from the GLP-1 receptor agonist, GIP/GLP-1 receptor agonist and GLP-1/GIP/GCG receptor triple agonist is contained in an amount of less than 3% by weight, it is not preferable because a single administration cannot provide long-term medicinal efficacy. In addition, if one or more selected from the GLP-1 receptor agonist, GIP/GLP-1 receptor agonist and GLP-1/GIP/GCG receptor triple agonist is contained in an amount exceeding 50% by weight, the initial release amount of the GLP-1 receptor agonist, GIP/GLP-1 receptor agonist or GLP-1/GIP/GCG receptor triple agonist in the body environment may be excessively high, causing a problem in that the blood concentration of the drug increases rapidly.

In addition, the content range of the biocompatible polymer contained in the microparticles may be 50 to 97% by weight based on the total weight of the microparticles. More specifically, the content of the biocompatible polymer may be 50% by weight or more, 55% by weight or more or 60% by weight or more, or may be 97% by weight or less, 96.0% by weight or less, 95.0% by weight or less, 94.0% by weight or less, 93.0% by weight or less, 92.0% by weight or less, 91.0% by weight or less or 90.0% by weight or less, based on the total weight of the microparticles, but is not limited to these ranges.

In the above, if the biocompatible polymer exceeds 97% by weight, the dissolution of the drug may become too slow, making it difficult to secure a consistent blood drug profile, and if the biocompatible polymer is contained in an amount of less than 50% by weight based on the total weight of the microspheres, the distribution of the GLP-1 receptor agonist, GIP/GLP-1 receptor agonist or GLP-1/GIP/GCG receptor triple agonist may relatively increase, resulting in problems such as initial excessive release or inability to maintain the medicinal efficacy for a desired period of time.

In one embodiment of the present invention, the microspheres may contain one or more selected from the GLP-1 receptor agonist, GIP/GLP-1 receptor agonist and GLP-1/GIP/GCG receptor triple agonist and the biocompatible polymer at a weight ratio of, for example, 1:1-50, 1:1.5-50, 1:1.6-50, 1:1.7-50, 1:1-47, 1:1.5-47, 1:1.6-47 1:1.7-47, 1:1-45, 1:1.5-45, 1:1.6-45, 1:1.7-45, 1:1-42, 1:1.5-42, 1:1.6-42, 1:1.7-42, 1:1-40, 1:1.5-40, 1:1.6-40, 1:1.7-40, 1:1-38, 1:1.5-38, 1:1.6-38, 1:1.7-38, 1:1-35, 1:1.5-35, 1:1.6-35 or 1:1.7-35.

In this case, if the weight ratio of the biocompatible polymer to one or more selected from the GLP-1 receptor agonist, GIP/GLP-1 receptor agonist and GLP-1/GIP/GCG receptor triple agonist is less than 1.0, it is not preferable because fatal side effects may occur due to initial excessive drug release, and if the weight ratio exceeds 50, the dissolution of the drug may become too slow, making it difficult to secure a constant blood drug profile.

In one embodiment of the present invention, the microspheres may have a dissolution rate of one or more selected from the GLP-1 receptor agonist, GIP/GLP-1 receptor agonist and GLP-1/GIP/GCG receptor triple agonist of 60% by weight or less, 55% by weight or less or 50% by weight or less within 7 days.

In addition, the microspheres may have a dissolution rate of one or more selected from the GLP-1 receptor agonist, GIP/GLP-1 receptor agonist and GLP-1/GIP/GCG receptor triple agonist of 25% by weight or less, 23% by weight or less or 20% by weight or less within 3 days.

In particular, the microspheres of the present invention may have a drug dissolution rate of one or more selected from the GLP-1 receptor agonist, GIP/GLP-1 receptor agonist and GLP-1/GIP/GCG receptor triple agonist of 10% by weight or less, 8% by weight or less or 5% by weight or less during the first day of administration.

In addition, the microspheres of the present invention may continuously release the drug for 10 days or more, 20 days or more, 1 month or more, 2 months or more, 3 months or more, 4 months or more, 5 months or more or 6 months or more.

In one embodiment of the present invention, the microspheres may be prepared by various microsphere preparation methods known in the art (e.g., O/W type, O/O type or W/O/W type solvent evaporation method or solvent extraction method, microsphere preparation method by spray drying, microsphere preparation method by phase separation, etc.).

In one embodiment of the present invention, the microspheres may be prepared by a solvent evaporation or extraction method through an emulsion, more preferably an oil-in-water (O/W) type solvent evaporation method, which comprises preparing an O/W type emulsion containing a biocompatible polymer; one or more selected from a GLP-1 receptor agonist, a GIP/GLP-1 receptor agonist and a GLP-1/GIP/GCG receptor triple agonist; and a dispersion solvent, and aggregating it into microspheres.

In order to prepare microspheres by the method for preparing the O/W type emulsion and aggregating it into polymer microparticles, first, an O/W type emulsion comprising a biocompatible polymer; one or more selected from a GLP-1 receptor agonist, a GIP/GLP-1 receptor agonist and a GLP-1/GIP/GCG receptor triple agonist; and a dispersion solvent is prepared.

The O/W type emulsion may be prepared using a conventional method known in the art, more specifically, by adding a dispersed phase containing one or more selected from a GLP-1 receptor agonist, a GIP/GLP-1 receptor agonist and a GLP-1/GIP/GCG receptor triple agonist, and a biocompatible polymer, to a dispersion solvent.

Such microspheres containing one or more selected from the GLP-1 receptor agonist, GIP/GLP-1 receptor agonist and GLP-1/GIP/GCG receptor triple agonist may be prepared by aggregating the emulsion into microspheres by the solvent extraction method and/or solvent evaporation method, or by aggregation through an ammonolysis process with the addition of ammonia or a hydrolysis process with the addition of an acid or base. A water-insoluble organic solvent that is converted into a water-soluble solvent by ammonolysis or hydrolysis reaction may be further included in the preparation of the emulsion.

In the case of the solvent evaporation method, polymer microspheres containing one or more selected from a GLP-1 receptor agonist, a GIP/GLP-1 receptor agonist and a GLP-1/GIP/GCG receptor triple agonist may be formed by, but not limited to, methods described in, for example, U.S. Patent Nos. 5,271,945, 5,985,309 and 6,471,996, i.e., by dissolving a polymer compound in an organic solvent phase, dispersing or dissolving a drug in the organic solvent phase, and subsequently emulsifying it in a dispersion medium such as water to prepare an O/W type emulsion, and then diffusing the organic solvent in the emulsion into the dispersion medium and evaporating it through an air/water interface.

The solvent extraction method includes conventional solvent extraction methods used in the preparation of microspheres containing one or more selected from a GLP-1 receptor agonist, a GIP/GLP-1 receptor agonist and a GLP-1/GIP/GCG receptor triple agonist, such as effectively extracting an organic solvent in emulsion droplets with a large amount of a solubilizing solvent.

As a method of simultaneously applying the solvent evaporation method and the solvent extraction method, for example, the methods described in U.S. Patent Nos. 4,389,840, 4,530,840, 6,368,632, 6,544,559 and 6,572,894 may be applied.

The aggregation by the ammonolysis process refers to a method of adding ammonia to an O/W type emulsion containing a water-insoluble organic solvent to induce ammonolysis, and converting the water-insoluble organic solvent into a water-soluble solvent to aggregate microspheres, such as the method described in, for example, Korean Patent No. 918092.

The aggregation by the hydrolysis process refers to a method of adding a base solution such as NaOH, LiOH or KOH, or an acid solution such as HCl or H2SO4 to an O/W type emulsion containing a water-insoluble organic solvent to induce hydrolysis, which is a type of hydrolysis reaction of an ester, and converting the water-insoluble organic solvent into a water-soluble solvent to aggregate microspheres, such as methods described in, for example, Korean Patent Application Nos. 2009-109809 and 2010-70407.

In one embodiment of the present invention, the microspheres may be W1/O/W2 (water-in-oil-in-water) type microspheres prepared by producing a W1/O (water-in-oil) type emulsion containing an inner water phase (W1) in which one or more selected from a GLP-1 receptor agonist, a GIP/GLP-1 receptor agonist and a GLP-1/GIP/GCG receptor triple agonist are dispersed or dissolved in an aqueous solvent; and an oil phase (O) in which a biocompatible polymer is dissolved in a non-aqueous solvent, and dispersing it in an outer water phase (W2). In this case, the microspheres may be prepared by a double emulsion evaporation method.

In order to prepare microspheres by the method of producing the W/O/W type emulsion and aggregating it into polymer microparticles, first, a W/O/W type emulsion comprising a biocompatible polymer, one or more selected from a GLP-1 receptor agonist, a GIP/GLP-1 receptor agonist and a GLP-1/GIP/GCG receptor triple agonist, and a dispersion solvent is prepared.

In this case, the W/O/W type emulsion may be prepared using a conventional method known in the art, more specifically, by adding a dispersed phase containing one or more selected from a GLP-1 receptor agonist, a GIP/GLP-1 receptor agonist and a GLP-1/GIP/GCG receptor triple agonist, and a biocompatible polymer, to a dispersion solvent.

Such microspheres containing one or more selected from the GLbnP-1 receptor agonist, GIP/GLP-1 receptor agonist and GLP-1/GIP/GCG receptor triple agonist may be prepared by aggregating the emulsion into microspheres by the solvent extraction method and/or solvent evaporation method, or by aggregation through an ammonolysis process with the addition of ammonia or a hydrolysis process with the addition of an acid or base. A water-insoluble organic solvent that is converted into a water-soluble solvent by ammonolysis or hydrolysis reaction may be further included in the preparation of the emulsion.

The solvent evaporation method, solvent extraction method, aggregation by ammonolysis process, and aggregation by hydrolysis process may be performed in the same manner as described above.

In one embodiment of the present invention, the pharmaceutical composition may further comprise one or more pharmaceutically acceptable additives. The additives may be one or more selected from the group consisting of, for example, a buffer, an isotonic agent, a preservative, and a pH adjusting agent.

The additives are distinct from the first carriers, and the pharmaceutical composition according to the present invention may be formulated in a suitable form comprising the microspheres, the pharmaceutically acceptable first carriers, and the additives.

The buffer may include, but is not limited to, for example, phosphate buffer, TRIS, citrate, etc. and any buffer known in the art may be used without limitation. The phosphate buffer may include sodium dihydrogen phosphate, disodium hydrogen phosphate, sodium phosphate, etc. The buffer may be included in the pharmaceutical composition, for example, at a concentration of 0.01-50 mM.

The isotonic agent may include, but is not limited to, for example, propylene glycol, sodium chloride, mannitol, sucrose, sorbitol, trehalose, lactose, etc. and any isotonic agent known in the art may be used without limitation. The isotonic agent may be included in the pharmaceutical composition, for example, at a concentration of 8 mg/ml to 50 mg/ml or at a concentration of 8 mg/ml to 30 mg/ml.

The preservative may include, but is not limited to, for example, phenol, etc., and any preservative known in the art may be used without limitation. The preservative may be included, for example, in an amount of 1% by weight or less, 0.5% by weight or less or 0.1% by weight or less, based on the total weight of the pharmaceutical composition.

The pH adjusting agent may include, but is not limited to, for example, HCl, NaOH, acetate, etc., and any pH adjusting agent known in the art may be used without limitation.

The pharmaceutical composition of the present invention may be formulated into various forms of preparations, for example, into a preparation for parenteral administration. The preparation for parenteral administration may include, but is not limited to, injections, creams, lotions, ointments for external use, oils, moisturizers, gels, aerosols, patches, nasal inhalers, etc.

The formulation for parenteral administration may further comprise, in addition to the microparticles, a thickener, a stabilizer, an isotonic agent, a pH adjusting agent, a surfactant, an excipient and/or a carrier.

The available isotonic agent may include water-soluble excipients or sugars such as mannitol, sucrose, sorbitol, trehalose, lactose and sodium chloride.

Examples of the thickener may include sodium carmellose, sodium carboxymethyl cellulose, povidone, etc.

The surfactant may include polyvinyl alcohol, etc. In addition, a buffering agent may include sodium hydrogen phosphate, anhydrous citric acid, sodium hydroxide, sodium chloride, etc.

The formulation for parenteral administration is not limited to the form exemplified above and may be formulated in a manner known in the art.

In one embodiment of the present invention, the pharmaceutical composition may be an injection.

In one embodiment of the present invention, the pharmaceutical composition may be an injection for subcutaneous or intramuscular injection.

In one embodiment of the present invention, the pharmaceutical composition may be administered to a subject at intervals of one month to one year.

In one embodiment of the present invention, the pharmaceutical composition may be used to prevent or treat diabetes, beta-cell function preservation, hypertension, hyperlipidemia, obesity, non-alcoholic steatohepatitis or degenerative neurological diseases such as Alzheimer's disease and Parkinson's disease.

The pharmaceutical composition of the present invention may be administered in a therapeutically effective amount of a GLP-1 receptor agonist, GIP/GLP-1 receptor agonist or GLP-1/GIP/GCG receptor triple agonist, for example, an amount effective for treating diabetes, specifically type 2 diabetes, beta-cell function preservation, hypertension, hyperlipidemia, obesity, non-alcoholic steatohepatitis or degenerative neurological diseases such as Alzheimer's disease and Parkinson's disease. The therapeutically effective amount of the GLP-1 receptor agonist, GIP/GLP-1 receptor agonist or GLP-1/GIP/GCG receptor triple agonist may be assessed by a physician.

The pharmaceutical composition of the present invention may be administered, but not limited to, once every two weeks, once every three weeks, once every month, once every three months, once every six months or once every year.

The pharmaceutical composition of the present invention may be administered, for example, by subcutaneous injection. The subcutaneous injection may be performed, for example, using a pre-filled syringe, for example, a pen syringe, which may be a disposable syringe containing a single dose, or a metered dose syringe (syringe with dose metering device) in which only a single dose is injected at a time.

The pharmaceutical composition of the present invention exhibits sufficient medicinal efficacy with one or more of a GLP-1 receptor agonist, a GIP/GLP-1 receptor agonist and a GLP-1/GIP/GCG receptor triple agonist during the treatment period, and therefore, it may be usefully employed in the prevention or treatment of diabetes, specifically type 2 diabetes, beta-cell function preservation, hypertension, hyperlipidemia, obesity, non-alcoholic steatohepatitis or degenerative neurological diseases such as Alzheimer's disease and Parkinson's disease.

The pharmaceutical composition of the present invention may be prepared, transported and stored in a state in which microspheres containing one or more selected from a GLP-1 receptor agonist, a GIP/GLP-1 receptor agonist and a GLP-1/GIP/GCG receptor triple agonist are suspended in a fluid oil carrier containing a fatty acid ester.

In addition, the pharmaceutical composition of the present invention may be prepared, transported and stored in a state in which microspheres containing one or more selected from a GLP-1 receptor agonist, a GIP/GLP-1 receptor agonist and a GLP-1/GIP/GCG receptor triple agonist are suspended in a carrier containing a surfactant or a polyhydric alcohol.

In addition, the pharmaceutical composition of the present invention may be prepared, transported and stored in a state in which microspheres containing one or more selected from a GLP-1 receptor agonist, a GIP/GLP-1 receptor agonist and a GLP-1/GIP/GCG receptor triple agonist are suspended in a carrier comprising a fluid oil containing a fatty acid ester, and a surfactant or a polyhydric alcohol.

The pharmaceutical composition of the present invention may be provided in a sealed container. The sealed container may be, for example, a vial (disposable or reusable), a syringe, an injection pen (e.g., disposable or reusable), etc.

The pharmaceutical composition of the present invention provides long-term storage stability. For example, the pharmaceutical composition of the present invention may be stored in a stable state for 6 months or more, 1 year or more, 1 year and 6 months or more, or 2 years or more upon storage. The "stable state" means a condition in which the activity of the drug may be maintained at least 90% or more, 95% or more or 99% or more, compared to its activity (i.e., 100%) in the initial formulation. In particular, it means that the particle size and/or shape of the microspheres are maintained, and accordingly, the activity of the drug is maintained within the above range.

The microspheres included in the pharmaceutical composition of the present invention may be prepared using a method known in the art, for example, a "solvent extraction and evaporation method".

Specifically, these may be prepared by, for example, a method comprising the steps of:
(a) dissolving one or more selected from a GLP-1 receptor agonist, a GIP/GLP-1 receptor agonist and a GLP-1/GIP/GCG receptor triple agonist, and a biocompatible polymer in an organic solvent to prepare a solution (dispersed phase) containing one or more selected from the GLP-1 receptor agonist, GIP/GLP-1 receptor agonist and GLP-1/GIP/GCG receptor triple agonist, and the polymer;
(b) adding the solution containing one or more selected from the GLP-1 receptor agonist, GIP/GLP-1 receptor agonist and GLP-1/GIP/GCG receptor triple agonist, and the polymer prepared in the step (a) to an aqueous solution phase (continuous phase) to prepare an emulsion;
(c) extracting and evaporating the organic solvent from the dispersed phase of the emulsion prepared in the step (b) into the continuous phase to form microspheres; and
(d) recovering the microspheres from the continuous phase in the step (c) to prepare microspheres containing one or more selected from the GLP-1 receptor agonist, GIP/GLP-1 receptor agonist and GLP-1/GIP/GCG receptor triple agonist.

One or more selected from the GLP-1 receptor agonist, GIP/GLP-1 receptor agonist and GLP-1/GIP/GCG receptor triple agonist, the biocompatible polymer, and the like used in the preparation method have been described above, and therefore, description thereof will be omitted in this section.

In the step (a), one or more selected from a GLP-1 receptor agonist, a GIP/GLP-1 receptor agonist and a GLP-1/GIP/GCG receptor triple agonist may be first dissolved in an organic solvent and then mixed with a biocompatible polymer solution.

In the step (a), an organic solvent that is miscible with water, or an organic solvent that is not miscible with water may be used as the organic solvent. Alternatively, a mixed solvent of the organic solvent that is miscible with water and the organic solvent that is not miscible with water may be used.

It is preferable to use the organic solvent that is not miscible with water in an amount of 50% (v/v) or more of the total solvent. If the property of the organic solvent not being miscible with water is utilized, a dispersed phase may be homogeneously mixed in a continuous phase to efficiently form an emulsion.

The type of the organic solvent that dissolves one or more selected from the GLP-1 receptor agonist, GIP/GLP-1 receptor agonist and GLP-1/GIP/GCG receptor triple agonist and the biocompatible polymer is not particularly limited, but preferably, one or two or more mixed solvents selected from the group consisting of dichloromethane, chloroform, ethyl acetate, methyl ethyl ketone, acetone, acetonitrile, dimethyl sulfoxide, dimethylformamide, N-methylpyrrolidone, acetic acid, methyl alcohol, ethyl alcohol, propyl alcohol and benzyl alcohol may be used.

In the step (b), the step of adding one or more selected from the GLP-1 receptor agonist, GIP/GLP-1 receptor agonist and GLP-1/GIP/GCG receptor triple agonist and the polymer-containing solution (dispersed phase) to an aqueous solution phase (continuous phase) to prepare an emulsion may be performed using a homogenizer such as a high-speed stirrer, an in-line mixer, a membrane emulsification method, a microfluidic emulsification method, an ultrasonic mixer or a static mixer.

In the step (b), the continuous phase may further contain a surfactant. The type of the surfactant is not particularly limited, and any one may be used as long as it can help one or more selected from the GLP-1 receptor agonist, GIP/GLP-1 receptor agonist and GLP-1/GIP/GCG receptor triple agonist and the polymer solution to form a stable droplet dispersed phase within the continuous phase. As the surfactant, a hydrophilic polymer selected from the group consisting of methylcellulose, polyvinylpyrrolidone, carboxymethylcellulose, lecithin, gelatin, polyvinyl alcohol, polyoxyethylene-polyoxypropylene block copolymer, polyoxyethylene sorbitan fatty acid ester and polyoxyethylene castor oil derivative, and mixtures thereof may be used, and particularly preferably, polyvinyl alcohol may be used.

The surfactant may be added to the continuous phase at a concentration of 0.1 to 3% (w/v), more preferably at a concentration of 0.3 to 2% (w/v). If the concentration of the surfactant is less than 0.1%, a dispersed phase or emulsion in the form of droplets may not be formed within the continuous phase, and if the concentration of the surfactant exceeds 3%, it may be difficult to remove the surfactant after the microspheres are formed.

Water may be used as the aqueous solution phase (continuous phase) in the step (b), and the pH of the aqueous solution phase may be, but is not limited to, 4.0 to 10. NaCl, which plays a role in osmotic regulation, may be used to control the initial release.

In the step (c), if the emulsion is maintained or stirred at a temperature below the boiling point of the organic solvent for a certain time, for example, 2 to 48 hours, the organic solvent may be extracted from the dispersed phase to the continuous phase. A portion of the organic solvent extracted into the continuous phase may be evaporated from the surface. In this case, the dispersed phase in the form of droplets may be solidified to form microspheres.

In order to efficiently remove the organic solvent in the step (c), the continuous phase may be heated for a certain time. The heating temperature is not limited and may be appropriately adjusted by those skilled in the art depending on the organic solvent used.

The method of recovering the microspheres in the step (d) may be performed using various known techniques, and for example, a method such as filtration or centrifugation may be used.

Between the steps (c) and (d), it is also possible to remove the remaining surfactant through filtration and washing, and filter again to recover the microspheres. The washing step to remove the remaining surfactant may be typically performed using water, and the washing step may be repeated several times.

The microspheres obtained after the step (d) may be dried using a conventional drying method to obtain the final dried microspheres.

The microspheres of the present invention may also be prepared by the following method.

There is provided a method for preparing microspheres, comprising the steps of:
(1) dissolving or dispersing one or more selected from a GLP-1 receptor agonist, a GIP/GLP-1 receptor agonist and a GLP-1/GIP/GCG receptor triple agonist in a solvent to prepare an inner water phase (W1);
(2) dissolving a biocompatible polymer in one or more solvents to prepare an oil phase (O);
(3) dispersing the inner water phase (W1) in the oil phase (O) to prepare a water-in-oil (W1/O) type emulsion;
(4) forming microspheres by dispersing the water-in-oil (W1/O) type emulsion into an outer continuous phase (W2) to prepare a water-in-oil-in-water (W1/O/W2) type emulsion; and
(5) removing the solvent.

Among the contents regarding the microspheres described above and the method for preparing the microspheres, all of the contents (type of biocompatible polymer, intrinsic viscosity thereof, etc.) that are commensurate with the preparation method may be applied to the method for preparing the microspheres of the present invention. Therefore, description of redundant contents will be omitted below.

The step (1) may be a step of dissolving, for example, one or more selected from a GLP-1 receptor agonist, a GIP/GLP-1 receptor agonist and a GLP-1/GIP/GCG receptor triple agonist in distilled water to prepare a water phase (W1).

In the step (2), the type of solvent is not particularly limited, and for example, dimethyl sulfoxide, dichloromethane or a mixed solvent thereof may be used. Particularly preferably, dichloromethane may be used.

The step (3) is a step of dispersing the inner water phase (W1) prepared in the step (1) in the oil phase (O) prepared in the step (2) to prepare a water-in-oil (W1/O) type emulsion.

In the step (3), the weight ratio of the oil phase (O) to the internal water phase (W1) may be 1:10 or less.

The step (4) is a step of solidifying microspheres by dispersing the water-in-oil (W1/O) type dispersed phase prepared in the step (3) into an outer continuous phase (W2) to prepare a water-in-oil-in-water (W1/O/W2) type emulsion solution.

In the step (4), the outer continuous phase (W2) may contain a hydrophilic polymer as a surfactant, and the type thereof is not particularly limited, and any one may be used as long as it can help the dispersed phase containing one or more selected from the GLP-1 receptor agonist, GIP/GLP-1 receptor agonist and GLP-1/GIP/GCG receptor triple agonist and the biocompatible polymer to form a stable droplet dispersed phase within the outer continuous phase.

In the step (4), the outer continuous phase may be an aqueous hydrophilic polymer solution of 0.1 to 6% (w/v), preferably 0.1 to 4% (w/v).

In the step (4), the dispersed phase (W1/O) containing one or more selected from the GLP-1 receptor agonist, GIP/GLP-1 receptor agonist and GLP-1/GIP/GCG receptor triple agonist and the biocompatible polymer prepared in the step (3) is added to the outer continuous phase containing the hydrophilic polymer in a drop-by-drop manner or in a manner using an in-line mixer, and stirred vigorously to prepare a water-in-oil-in-water (W1/O/W2) type emulsion solution.

Afterwards, in the step (5), the solvent is removed, and conventional filtration and washing are performed, and then the desired microspheres may be obtained. That is, a step of washing the obtained microspheres using an organic solvent such as ethanol may be included to improve the initial release-inhibiting effect as needed.

Hereinafter, the present invention will be described in detail by way of examples in order to specifically explain the present invention. However, the examples according to the present invention may be modified in various different forms, and the scope of the present invention should not be construed as being limited to the examples described below. The examples of the present invention are provided to more fully describe the present invention to a person having ordinary skill in the art.

### Preparative Example 1: Preparation of semaglutide microspheres

### <Preparation of continuous phase>

900 ml of distilled water was placed in the reactor, and 4.5 g of polyvinyl alcohol (PVA) and 4.5 g of sodium chloride (NaCl) were weighed and placed therein. It was stirred at 1,000 rpm using a homogenizer (Overhead, IKA) to prepare a 0.5% (w/v) aqueous polyvinyl alcohol solution.

### <Preparation of semaglutide solution>

0.32 g of semaglutide and 2.4 ml of distilled water were weighed and placed in a 20 ml vial, and stirred at 150 rpm to dissolve. The solution prepared above was stored in a refrigerator.

### <Preparation of PLGA solution>

2.88 g of PLGA (intrinsic viscosity of 0.15 dL/g to 1.7 dL/g) was added to 9.6 ml of dichloromethane, and stirred at 300 rpm to dissolve.

### <Emulsification>

The PLGA solution prepared above was taken into a syringe, added to the semaglutide solution, and stirred at 20,000 rpm for 1 minute using a homogenizer (IKA).

### <Recovery of microspheres>

After the emulsification process was completed, the solution was immediately taken into a syringe and placed into a 1 L reactor in which the continuous phase was previously introduced, while stirring using a homogenizer (Kinematica) for 60 seconds. Microspheres were then solidified for 20 hours. After solidification was completed, the microspheres (W/O/W type) were obtained by filtering through a 5 µm filter. The obtained microspheres were then freeze-dried in a freeze dryer for 20 hours or more.

### Preparative Example 2: Preparation of semaglutide microspheres

### <Preparation of continuous phase>

900 ml of distilled water was placed in the reactor, and 4.5 g of polyvinyl alcohol (PVA) and 4.5 g of sodium chloride (NaCl) were weighed and placed therein. It was stirred at 1,000 rpm using a homogenizer (Overhead, IKA) to prepare a 0.5% (w/v) aqueous polyvinyl alcohol solution.

### <Preparation of semaglutide solution>

0.48 g of semaglutide and 2.4 ml of distilled water were weighed and placed in a 20 ml vial, and stirred at 150 rpm to dissolve.

### <Preparation of PLGA solution>

1.44 g of PLGA (intrinsic viscosity of 0.15 dL/g to 1.7 dL/g) was added to 4.8 ml of dichloromethane, and stirred at 300 rpm to dissolve.

### <Emulsification>

The PLGA solution prepared above was taken into a syringe, added to the semaglutide solution, and stirred at 20,000 rpm for 1 minute using a homogenizer (IKA).

### <Recovery of microspheres>

After the emulsification process was completed, the solution was immediately taken into a syringe and placed into a 1 L reactor in which the continuous phase was previously introduced, while stirring using a homogenizer (Kinematica) for 60 seconds. Microspheres were then solidified for 20 hours. After solidification was completed, the microspheres (W/O/W type) were obtained by filtering through a 5 µm filter. The obtained microspheres were then freeze-dried in a freeze dryer for 20 hours or more.

### Example 1: Preparation of an injectable liquid formulation comprising semaglutide microspheres and MCT oil

Semaglutide microspheres of Preparative Example 1 corresponding to 3 mg/kg based on semaglutide were weighed and placed in a vial, and then MCT oil was placed therein and suspended to prepare a semaglutide microsphere injection.

### Example 2: Preparation of an injectable liquid formulation comprising semaglutide microspheres and castor oil

A semaglutide microsphere injection was prepared in the same manner as in Example 1, except that the MCT oil was replaced with castor oil.

### Example 3: Preparation of an injectable liquid formulation comprising semaglutide microspheres and cottonseed oil

A semaglutide microsphere injection was prepared in the same manner as in Example 1, except that the MCT oil was replaced with cottonseed oil.

### Example 4: Preparation of an injectable liquid formulation comprising semaglutide microspheres and sesame oil

A semaglutide microsphere injection was prepared in the same manner as in Example 1, except that the MCT oil was replaced with sesame oil in Example 1.

### Example 5: Preparation of an injectable liquid formulation comprising semaglutide microspheres and Polyoxyl 35 castor oil

A semaglutide microsphere injection was prepared in the same manner as in Example 1, except that the MCT oil was replaced with Polyoxyl 35 castor oil in Example 1.

### Example 6: Preparation of an injectable liquid formulation comprising semaglutide microspheres and a liquid propylene glycol carrier

The microspheres prepared in the Preparative Example 2 were weighed so that the dosage of semaglutide would be 1 mg/kg, and then propylene glycol was added as a carrier, and these were mixed to prepare a semaglutide microsphere injection.

### Example 7: Preparation of an injectable liquid formulation comprising semaglutide microspheres and MCT oil

A semaglutide microsphere injection was prepared in the same manner as in Example 6, except that the propylene glycol was replaced with MCT oil.

### Comparative Example 1: Preparation of an injectable liquid formulation comprising semaglutide microspheres and a diluent.

A semaglutide microsphere injection was prepared in the same manner as in Example 1, except that the MCT oil was replaced with a diluent containing citric acid anhydrous, sodium monohydrogen phosphate dihydrate, sodium carboxymethylcellulose, polysorbate 20, sodium chloride and sodium hydroxide.

### Comparative Example 2: Preparation of an injectable liquid formulation comprising semaglutide microspheres and a diluent.

A semaglutide microsphere injection was prepared in the same manner as in Example 6, except that the propylene glycol was replaced with a diluent containing citric acid anhydrous, sodium monohydrogen phosphate dihydrate, sodium carboxymethylcellulose, polysorbate 20, sodium chloride and sodium hydroxide.

### Preparative Example 3: Preparation of tirzepatide microspheres

### <Preparation of continuous phase>

900 ml of distilled water was placed in the reactor, and 4.5 g of polyvinyl alcohol (PVA) and 4.5 g of sodium chloride (NaCl) were weighed and placed therein. It was stirred at 1,000 rpm using a homogenizer (Overhead, IKA) to prepare a 0.5% (w/v) aqueous polyvinyl alcohol solution.

### <Preparation of tirzepatide solution>

0.3 g of tirzepatide and 1.0 ml of distilled water were weighed and placed in a 20 ml vial, and stirred at 150 rpm to dissolve.

### <Preparation of PLGA solution>

1.2 g of PLGA (intrinsic viscosity of 0.15 dL/g to 1.7 dL/g) was added to 3 ml of dichloromethane, and stirred at 300 rpm to dissolve. The solution prepared above was stored in a refrigerator.

### <Emulsification>

The PLGA solution prepared above was taken into a syringe, added to the tirzepatide solution, and stirred at 20,000 rpm for 1 minute using a homogenizer (IKA).

### Preparative Example 4: Preparation of tirzepatide microspheres

### <Preparation of continuous phase>

900 ml of distilled water was placed in the reactor, and 4.5 g of polyvinyl alcohol (PVA) and 4.5 g of sodium chloride (NaCl) were weighed and placed therein. It was stirred at 1,000 rpm using a homogenizer (Overhead, IKA) to prepare a 0.5% (w/v) aqueous polyvinyl alcohol (PVA) solution.

### <Preparation of tirzepatide solution>

0.386 g of tirzepatide and 1.5 ml of distilled water were weighed and placed in a 20 ml vial, stirred at 150 rpm to dissolve, and then 0.037 ml of propylene glycol was placed therein and stirred to dissolve.

### <Preparation of PLGA solution>

0.9 g of PLGA (intrinsic viscosity of 0.15 dL/g to 1.7 dL/g) was added to 3 ml of dichloromethane, and stirred at 300 rpm to dissolve.

### <Emulsification>

The PLGA solution prepared above was taken into a syringe, added to the tirzepatide solution, and stirred at 20,000 rpm for 1 minute using a homogenizer (IKA).

### <Recovery of microspheres>

After the emulsification process was completed, the solution was immediately taken into a syringe and placed into a 1 L reactor in which the continuous phase was previously introduced, while stirring using a homogenizer (Kinematica) for 60 seconds. Microspheres were then solidified for 20 hours. After solidification was completed, the microspheres (W/O/W type) were obtained by filtering through a 5 µm filter. The obtained microspheres were then freeze-dried in a freeze dryer for 20 hours or more.

### Example 8: Preparation of an injectable liquid formulation comprising tirzepatide microspheres and MCT oil

Tirzepatide microspheres of Preparative Example 3 corresponding to 3 mg/kg based on tirzepatide were weighed and placed in a vial, and then MCT oil was placed therein and suspended to prepare a tirzepatide microsphere injection.

### Example 9: Preparation of an injectable liquid formulation comprising tirzepatide microspheres and castor oil

A tirzepatide microsphere injection was prepared in the same manner as in Example 8, except that the MCT oil was replaced with castor oil in Example 8.

### Example 10: Preparation of an injectable liquid formulation comprising tirzepatide microspheres and cottonseed oil

A tirzepatide microsphere injection was prepared in the same manner as in Example 8, except that the MCT oil was replaced with cottonseed oil in Example 8.

### Example 11: Preparation of an injectable liquid formulation comprising tirzepatide microspheres and sesame oil

A tirzepatide microsphere injection was prepared in the same manner as in Example 8, except that the MCT oil was replaced with sesame oil in Example 8.

### Example 12: Preparation of an injectable liquid formulation comprising tirzepatide microspheres and Polyoxyl 35 castor oil

A tirzepatide microsphere injection was prepared in the same manner as in Example 8, except that the MCT oil was replaced with Polyoxyl 35 castor oil in Example 8.

### Example 13: Preparation of an injectable liquid formulation comprising tirzepatide microspheres and a liquid propylene glycol carrier

The microspheres prepared in the Preparative Example 4 were weighed so that the dosage of tirzepatide would be 1 mg/kg, and then propylene glycol was added as a carrier, and these were mixed to prepare a tirzepatide microsphere injection.

### Comparative Example 3: Preparation of an injectable liquid formulation comprising tirzepatide microspheres and a diluent

A tirzepatide microsphere injection was prepared in the same manner as in Example 8, except that the MCT oil was replaced with a diluent containing citric acid anhydrous, sodium monohydrogen phosphate dihydrate, sodium carboxymethylcellulose, polysorbate 20, sodium chloride and sodium hydroxide.

### Comparative Example 4: Preparation of injectable liquid formulation of tirzepatide microspheres

A tirzepatide microsphere injection was prepared in the same manner as in Example 13, except that the propylene glycol was replaced with a diluent containing citric acid anhydrous, sodium monohydrogen phosphate dihydrate, sodium carboxymethylcellulose, polysorbate 20, sodium chloride and sodium hydroxide in Example 13.

### Experimental Example 1: In vivo pharmacokinetics test using beagles

Each of the injectable formulation of semaglutide microspheres (Comparative Example 2) and the injection containing semaglutide microspheres and propylene glycol (Example 6) was administered to a beagle, and then the concentration of semaglutide in the blood was measured.

Specifically, each sample was injected subcutaneously into the beagle using a 23-gauge syringe, blood was collected at designated times, and the concentration of semaglutide in the blood was measured using LC-MS/MS. The change in the concentration of semaglutide in the blood is shown in FIG. 1.

### Experimental Example 2: In vivo pharmacokinetics test using beagles

Each of the injectable formulation of tirzepatide microspheres (Comparative Example 4) and the injection containing tirzepatide microspheres and propylene glycol (Example 13) was administered to a beagle, and then the concentration of semaglutide in the blood was measured.

Specifically, each sample was injected subcutaneously into the beagle using a 23-gauge syringe, blood was collected at designated times, and the concentration of semaglutide in the blood was measured using LC-MS/MS.

## Claims

1. A pharmaceutical composition, comprising:
microspheres containing one or more selected from a GLP-1 receptor agonist, a GIP/GLP-1 receptor agonist and a GLP-1/GIP/GCG receptor triple agonist; and
one or more pharmaceutically acceptable first carriers selected from the group consisting of a fluid oil containing a fatty acid ester, a surfactant and a polyhydric alcohol.

2. The pharmaceutical composition of claim 1, wherein the fluid oil is one or more selected from coconut oil, palm oil, palm kernel oil, sesame oil, soybean oil, almond oil, rapeseed oil, corn oil, sunflower oil, peanut oil, olive oil, castor oil, safflower oil, cottonseed oil and ethyl oleate.

3. The pharmaceutical composition of claim 1, wherein the surfactant is one or more selected from poloxamer 188, poloxamer 407, polysorbate 20, polysorbate 60, polysorbate 80, polyoxyethylene oleyl ether, polyoxyethylene cetyl ether, polyoxyethylene stearyl ether and polyoxyethylene lauryl ether.

4. The pharmaceutical composition of claim 1, wherein the polyhydric alcohol is one or more selected from ethylene glycol, propylene glycol, polyethylene glycol, glycerol, erythritol, threitol, xylitol, ribitol, mannitol, sorbitol and maltitol.

5. The pharmaceutical composition of claim 4, wherein the polyhydric alcohol is ethylene glycol, propylene glycol, polyethylene glycol or glycerol.

6. The pharmaceutical composition of claim 1, wherein the pharmaceutical composition comprises one or more selected from the GLP-1 receptor agonist, GIP/GLP-1 receptor agonist and GLP-1/GIP/GCG receptor triple agonist contained in the microspheres in a concentration ranging from 0.01 mg to 2,000 mg based on 1 mL of the first carriers.

7. The pharmaceutical composition of claim 1, wherein the GLP-1 receptor agonist, GIP/GLP-1 receptor agonist and/or GLP-1/GIP/GCG receptor triple agonist are one or more selected from exenatide, liraglutide, lixisenatide, albiglutide, dulaglutide, semaglutide, tirzepatide, cotadutide, taspoglutide, retatrutide and pharmaceutically acceptable salts thereof.

8. The pharmaceutical composition of claim 1, wherein one or more selected from the GLP-1 receptor agonist, GIP/GLP-1 receptor agonist and GLP-1/GIP/GCG receptor triple agonist are contained in an amount of 3 to 50% by weight based on the total weight of the microspheres.

9. The pharmaceutical composition of claim 1, wherein the microspheres further contain a biocompatible polymer.

10. The pharmaceutical composition of claim 9, wherein the biocompatible polymer is one or more selected from polylactic acid, polylactide, polylactic-co-glycolic acid, polylactide-co-glycolide (PLGA), polyphosphazine, polyiminocarbonate, polyphosphoester, polyanhydride, polyorthoester, copolymer of lactic acid and caprolactone, polycaprolactone, polyhydroxyvalerate, polyhydroxybutyrate, polyamino acid, and copolymer of lactic acid and amino acid.

11. The pharmaceutical composition of claim 9, wherein the biocompatible polymer is contained in an amount of 50 to 97% by weight based on the total weight of the microparticles.

12. The pharmaceutical composition of claim 1, wherein the pharmaceutical composition further comprises one or more additives selected from a buffer, an isotonic agent, a preservative and a pH adjusting agent.

13. The pharmaceutical composition of claim 9, wherein the microspheres contain one or more selected from the GLP-1 receptor agonist, GIP/GLP-1 receptor agonist and GLP-1/GIP/GCG receptor triple agonist and the biocompatible polymer at a weight ratio of 1:1-50.

14. The pharmaceutical composition of claim 1, wherein the microspheres remain stable for 6 months or more.

15. The pharmaceutical composition of claim 1, wherein the pharmaceutical composition is an injection.
